# FASCICULE DE BREVET EUROPEEN

(11) **EP 1 633 261 B1**
(45) Date de publication et mention de la délivrance du brevet: **22.11.2006**
(21) Numéro de dépôt: 04767365.2
(22) Date de dépôt: 17.06.2004
(51) Int. Cl.: A61B 17/86

(54) **DISPOSITIF D'ANTI-EXTRACTION D' UNE VIS D'ANCRAGE POUR UN ELEMENT D' OSTEOSYNTHESE**
VORRICHTUNG ZUR PRÄVENTION DER ENTFERNUNG EINER VERANKERUNGSSCHRAUBE FÜR EIN OSTEOSYNTHESEELEMENT
DEVICE FOR PREVENTING THE REMOVAL OF AN ANCHORING SCREW FOR AN OSTEOSYNTHETIC ELEMENT

(30) Priorité: 17.06.2003 FR 0307280
(43) Date de publication de la demande: 15.03.2006
(73) Titulaire: SCIENT'X, 78284 Guyancourt (FR)
(72) Inventeur: CARLI, Olivier, CH-1207 Genève (CH); BEN-MOKHTAR, Mourad, F-75018 Paris (FR)
(74) Mandataire: Thibault, Jean-Marc
(86) Numéro de dépôt international: PCT/FR2004/001503
(87) Numéro de publication internationale: WO 2004/112627

(56) Documents cités:
- DE-A- 10 101 267
- GB-A- 2 323 533
- US-B1- 6 331 179

## Description

La présente invention concerne le domaine technique de l'ostéosynthèse au sens général et, en particulier, du rachis et elle vise, plus précisément, un dispositif d'anti-extraction d'une vis d'ancrage d'un élément d'ostéosynthèse au sens général, tel qu'une plaque par exemple.

Dans l'état de la technique, il est connu de mettre en oeuvre une plaque d'ostéosynthèse rigide, adaptée pour recouvrir, au moins partiellement, deux vertèbres consécutives, afin de les solidariser ensemble. Cette plaque d'ostéosynthèse est pourvue de trous ou d'alésages traversants, conçus pour recevoir chacun une vis destinée à être ancrée dans le corps des vertèbres.

Après l'implantation de tels dispositifs d'ostéosynthèse chez des patients, il a été constaté un phénomène d'avulsion ou d'extraction partielle des vis d'ancrage. Il en résulte une perte de stabilité de la plaque et un risque de lésion ou de blessure, notamment des organes placés à proximité.

Il apparaît donc le besoin d'assurer le blocage des vis d'ancrage, afin d'éviter qu'elles ressortent de la plaque d'ostéosynthèse.

Pour tenter de satisfaire ce besoin, l'état de la technique a proposé diverses solutions techniques. Par exemple, la demande de brevet FR 2 540 321 a décrit un dispositif d'ostéosynthèse comportant, en tant que moyens d'anti-extraction des vis d'ancrage, une contre-plaque rigide destinée à être fixée sur la face antérieure de la plaque d'ostéosynthèse. Cette contre-plaque est conformée pour présenter une forme sensiblement analogue à celle de la plaque d'ostéosynthèse, de manière à recouvrir totalement l'ensemble des têtes des vis d'ancrage. La contre-plaque est fixée sur la plaque d'ostéosynthèse à l'aide d'une vis adaptée pour se visser dans un taraudage, aménagé sur la plaque d'ostéosynthèse. La contre-plaque constitue, ainsi, une butée pour les têtes des vis.

Il doit être considéré que la mise en place de ces moyens d'anti-extraction représente une opération relativement longue à mener à bien, en raison de la phase de positionnement de la contre-plaque, suivie par une phase de vissage de la contre-plaque sur la plaque d'ostéosynthèse. Par ailleurs, la vis de fixation de la contre-plaque de la plaque d'ostéosynthèse risque de se dévisser entraînant un écartement de la contre-plaque par rapport à la plaque, ainsi qu'une avulsion des vis d'ancrage. De plus, il doit être considéré que l'utilisation d'une contre-plaque rigide présente un encombrement relativement important, susceptible d'entraîner des blessures ou des lésions.

L'objet de l'invention vise donc à remédier aux inconvénients énoncés ci-dessus en proposant un dispositif permettant l'anti-extraction d'une vis d'ancrage pour un élément d'ostéosynthèse, conçu pour présenter un moyen de blocage sûr et efficace de la vis d'ancrage, tout en présentant une facilité et une rapidité de mise en place.

Pour atteindre un tel objectif, l'objet de l'invention concerne un dispositif d'anti-extraction d'une vis d'ancrage pour un élément d'ostéosynthèse, un tel dispositif comportant :
■ une vis d'ancrage comprenant une tête prolongée par un fût fileté,
■ et un élément d'ostéosynthèse présentant un alésage de passage pour la vis d'ancrage.
Selon l'invention :
■ la vis d'ancrage comporte, au niveau de sa tête, au moins deux bras de blocage montés sur la vis et de manière élastique, pour occuper une position d'escamotage sous l'action d'un effort radial et une position de blocage sous l'action d'un effort radial inférieur,
■ et l'élément d'ostéosynthèse comporte, au niveau de son alésage, une section de sollicitation radiale pour les bras de blocage, débouchant en aval en considération du sens d'engagement de la vis, dans un canal de verrouillage de manière que lors de l'opération de vissage de la vis d'ancrage, les bras de blocage viennent occuper d'abord leur position d'escamotage en raison de l'action exercée par la section de sollicitation, puis leur position de blocage en prenant place dans le canal de verrouillage.

Selon une forme préférée de réalisation, chaque bras de blocage est constitué d'une tige mobile, fixée sur la vis en s'étendant, au repos, sensiblement parallèlement à l'axe de la vis d'ancrage et muni d'un ergot de blocage situé à l'opposé du point de fixation du bras de blocage.

De préférence, chaque bras de blocage est monté pour s'étendre en relation d'un dégagement, aménagé axialement sur la vis d'ancrage pour permettre aux bras de blocage d'occuper leur position d'escamotage.

Selon une caractéristique avantageuse de l'invention la position de montage des bras de blocage sur la vis d'ancrage et la position du canal de verrouillage sur l'élément d'ostéosynthèse sont telles que la vis d'ancrage occupe sensiblement sa position d'introduction finale lorsque les bras de blocage occupent leur position de blocage.

Selon une caractéristique préférée de réalisation, chaque bras de blocage comporte un prolongement axial de préhension, faisant saillie par rapport à la tête de la vis d'ancrage.

Avantageusement, la tête de la vis d'ancrage est équipée d'un moyen d'entraînement en rotation.

Un autre objet de l'invention est de proposer un instrument pour le démontage d'une vis d'ancrage faisant partie d'un dispositif d'anti-extraction conforme à l'invention. Cet instrument comporte un manche équipé :
■ d'un moyen de coopération avec le moyen d'entraînement en rotation de la vis d'ancrage,
■ et d'un moyen de déblocage adapté pour exercer un effort radial sur les bras de blocage, afin de les amener à occuper leur position d'escamotage.

Avantageusement, le moyen de déblocage exerce un effort radial sur les prolongements de préhension des bras de blocage.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est une vue, en perspective partielle, montrant un dispositif d'anti-extraction conforme à l'invention.

La **fig. 2** est une vue, en coupe-élévation, montrant un dispositif d'extraction en position montée.

La **fig. 3** est une vue, en coupe-élévation, montrant un dispositif d'anti-extraction associé à un instrument d'enlèvement de la vis d'ancrage.

Tel que cela apparaît sur les figures, l'invention concerne un dispositif **1** assurant l'anti-extraction d'une vis **2** d'ancrage osseux, permettant la fixation d'un élément d'ostéosynthèse **3**, au sens général, tel qu'une plaque dans l'exemple illustré. La vis d'ancrage **2** comprend une tête **4** prolongée par un fût fileté **5**. De manière classique, la tête **4** est équipée d'un moyen **7** d'entraînement en rotation. Par exemple, ce moyen d'entraînement en rotation **7** peut être constitué par un logement prismatique s'ouvrant sur la face supérieure **8** de la tête de la vis.

Selon une caractéristique avantageuse de l'invention, la vis d'ancrage **2** comporte, au niveau de sa tête **4**, au moins deux bras de blocage **11**, montés dans l'exemple illustré de façon diamétralement opposée, sur la vis **2.** Les bras de blocage **11** sont montés, de manière élastique, pour occuper une position d'escamotage sous l'action d'un effort radial et une position de blocage sous l'action d'un effort radial de valeur inférieure par rapport à celui entraînant leur placement dans la position d'escamotage. Il est à noter que dans la position de blocage, l'effort radial exercé sur les bras de blocage **11** peut être de valeur nulle ou non.

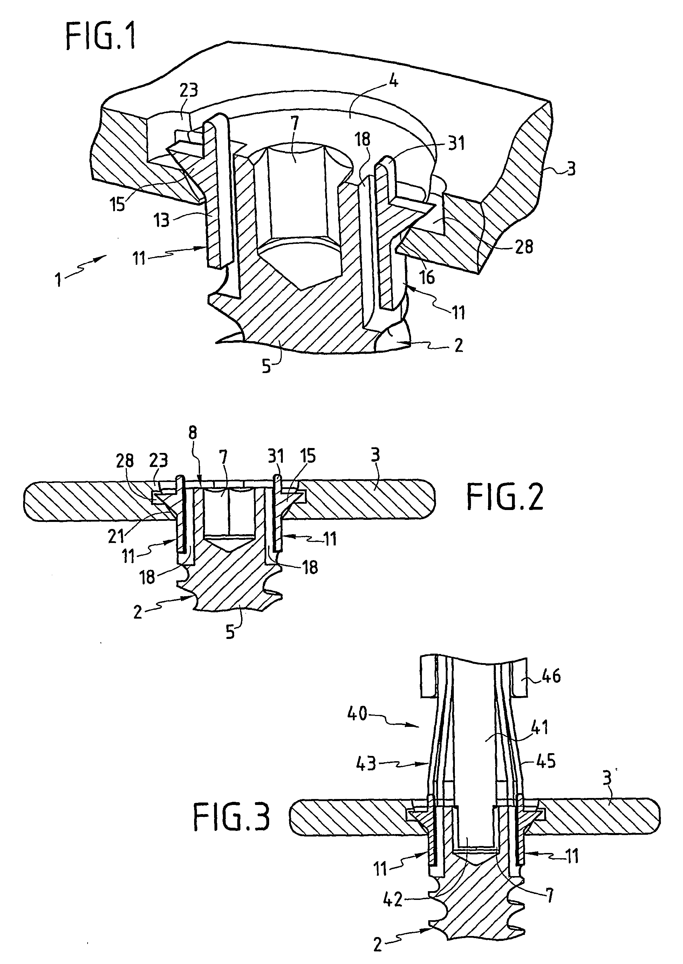

Tel que cela ressort plus précisément de la **fig. 1**, chaque bras de blocage **11** est constitué, par exemple, d'une tige mobile **13** fixée sur la vis en s'étendant au repos, c'est-à-dire sans subir l'action de forces extérieures, sensiblement parallèlement à l'axe de la vis d'ancrage **2**. Chaque bras de blocage **11** est muni, à l'opposé de son point de fixation, d'un ergot de blocage **15**, dont la fonction apparaîtra plus précisément dans la suite de la description.

De préférence, chaque ergot de blocage **15** s'étend radialement, à partir de la tige mobile **13**, en présentant, du côté tourné vers l'extrémité libre de la vis **2**, une rampe d'engagement **16** inclinée en direction de l'extrémité libre de la vis. Ainsi, dans la position de repos, les ergots de blocage **15** s'étendent en saillie par rapport au fût fileté **5** de la vis d'ancrage **2**.

Chaque bras de blocage **11** est monté pour s'étendre, en relation d'un dégagement **18** aménagé axialement sur la vis d'ancrage **2**, pour permettre aux bras de blocage **11** d'occuper leur position d'escamotage. Il doit être compris que l'application d'un effort radial sur les bras de blocage **11** permet de les rapprocher diamétralement, afin qu'ils occupent une largeur réduite par rapport à la largeur occupée dans leur position de blocage ou de repos.

Selon une autre caractéristique avantageuse de l'invention, l'élément d'ostéosynthèse **3** comporte au moins un trou ou alésage de passage **21** pour la vis d'ancrage **2**. Cet alésage de passage **21** comporte une section **23** de sollicitation radiale pour les bras de blocage **11**, de manière à amener les bras dans leur position d'escamotage. Il doit être compris que la section **23** présente une dimension inférieure à la largeur délimitée entre les bras de blocage **11**, lorsque ces derniers occupent leur position de blocage ou de repos. Bien entendu, cette section **23** possède une dimension adaptée pour autoriser le passage de la vis d'ancrage **2**. Cette section de sollicitation radiale **23** débouche en aval, en considération du sens d'engagement de la vis **2,** dans un canal **28** de verrouillage dans lequel les bras de blocage **11** occupent leur position de blocage. Il doit donc être compris que le canal de verrouillage **28** présente une dimension radiale supérieure à la section **23** et adaptée pour permettre, aux bras de blocage **11**, d'occuper leur position de repos. Dans cette position d'engagement des bras de blocage **11** à l'intérieur du canal de verrouillage **28**, la vis d'ancrage **2** ne peut pas être desserrée par rapport à l'élément d'ostéosynthèse **3** puisqu'un tel desserrage est empêché par la mise en butée des bras de blocage **11** sur le fond du canal de verrouillage **28** délimité par la section radiale **23**. De préférence, le canal de verrouillage **28** est réalisé par une rainure s'étendant sur toute la périphérie de l'alésage de passage **21**.

Le fonctionnement du dispositif d'anti-extraction **1** conforme à l'invention découle directement de la description qui précède. Après le positionnement de l'élément d'ostéosynthèse **3**, une vis d'ancrage **2** est mise en place en traversant l'alésage **21** aménagé dans l'élément d'ostéosynthèse **3**. Dans la phase finale de l'opération de vissage de la vis **2**, les bras de blocage **11** sont sollicités radialement en rapprochement par la section de sollicitation **23** en vue d'occuper leur position d'escamotage. La poursuite de l'opération de vissage de la vis **2** amène les bras de blocage **11** et plus précisément les ergots **15** à venir s'engager dans le canal de verrouillage **28** de sorte que les bras de blocage **11** s'écartent radialement pour occuper leur position de blocage. Il est à noter que dans cette position d'engagement, les bras de blocage **11** peuvent ou non subir une sollicitation radiale. Les bras de blocage **11** sont ainsi encliquetés dans le canal de verrouillage **28** de sorte que la vis d'ancrage **2** se trouve verrouillée dans les deux sens, avec l'élément d'ostéosynthèse **3**. Il est à noter et tel que cela ressort plus précisément de la **fig. 3**, la position de montage des bras de blocage **11** sur la vis d'ancrage **2** et la position du canal de verrouillage **28** sur l'élément d'ostéosynthèse **3** est telle que la vis d'ancrage **2** occupe sensiblement sa position d'introduction finale, lorsque les bras de blocage **11** sont dans leur position de blocage. Dans cette position de montage, la face supérieure **8** de la tête de la vis d'ancrage s'étend sensiblement en retrait par rapport à la face externe de l'élément d'ostéosynthèse **3**.

Dans la description qui précède, la vis d'ancrage **2** comporte deux bras de blocage **11** s'étendant de façon diamétralement opposée. Bien entendu, la vis d'ancrage **2** peut comporter un nombre différent de bras de blocage disposés de manière appropriée à la périphérie de la vis d'ancrage. Dans le même sens, les bras de blocage peuvent s'étendre selon une plage périphérique plus ou moins grande.

Selon une caractéristique préférée de l'invention, chaque bras de blocage **11** comporte un prolongement axial de préhension **31** faisant saillie par rapport à la tête de la vis d'ancrage et, notamment, par rapport à la surface supérieure **8**. De tels prolongements **31** peuvent, avantageusement, être sollicités en rapprochement par l'intermédiaire d'un instrument **40** pour permettre, en cas de besoin, le retrait de la vis d'ancrage **2**. Cet instrument **40** comporte un manche non représenté, prolongé par une tige **41** équipée d'un moyen **42** de coopération avec le moyen d'entraînement en rotation **7** de la vis d'ancrage **2**. Un tel instrument **40** est également équipé d'un moyen de déblocage **43** adapté pour exercer un effort radial sur les bras de blocage **11**, afin de les amener à occuper leur position d'escamotage de manière à autoriser l'opération de dévissage.

Dans l'exemple illustré, un tel moyen de déblocage **43** est constitué par l'intermédiaire de deux doigts mobiles **45**, aptes à être rapprochés l'un par rapport à l'autre radialement, à l'aide d'un fourreau **46** monté coulissant axialement pour permettre le rapprochement des doigts mobiles **45**, afin que ceux-ci exercent un effort radial sur les prolongements **31** de préhension des bras de blocage **11**. Dans cette position, l'instrument **40** peut alors être utilisé pour entraîner en rotation la vis d'ancrage dans son sens de desserrage, afin d'être démontée par rapport à l'élément d'ostéosynthèse **3**. Un tel retrait est possible dans la mesure où, pendant cette opération de desserrage les bras de blocage **11** sont maintenus dans leur position d'escamotage pour passer au-delà de la section de sollicitation radiale **23**.

L'invention n'est pas limitée aux exemples décrits et représentés car diverses modifications peuvent y être apportées sans sortir de son cadre.

## Revendications

1. Dispositif d'anti-extraction d'une vis d'ancrage pour un élément d'ostéosynthèse (**3**), un tel dispositif comportant :
■ une vis d'ancrage (**2**) comprenant une tête (**4**) prolongée par un fût fileté (**5**),
■ et un élément d'ostéosynthèse (**3**) présentant un alésage de passage (**21**) pour la vis d'ancrage,
**caractérisé en ce que**:
■ la vis d'ancrage (**2**) comporte, au niveau de sa tête, au moins deux bras de blocage (**11**) montés sur la vis et de manière élastique, pour occuper une position d'escamotage sous l'action d'un effort radial et une position de blocage sous l'action d'un effort radial de valeur inférieure,
■ et l'élément d'ostéosynthèse (**3**) comporte, au niveau de son alésage, une section (**23**) de sollicitation radiale pour les bras de blocage, débouchant en aval en considération du sens d'engagement de la vis, dans un canal de verrouillage (**28**) de manière que lors de l'opération de vissage de la vis d'ancrage (**2**), les bras de blocage (**11**) viennent occuper d'abord leur position d'escamotage en raison de l'action exercée par la section de sollicitation (**23**), puis leur position de blocage en prenant place dans le canal de verrouillage (**28**).

2. Dispositif d'anti-extraction selon la revendication 1, **caractérisé en ce que** chaque bras de blocage (**11**) est constitué d'une tige mobile (**13**), fixée sur la vis (**2**) en s'étendant, au repos, sensiblement parallèlement à l'axe de la vis d'ancrage et muni d'un ergot de blocage (**15**) situé à l'opposé du point de fixation du bras de blocage.

3. Dispositif d'anti-extraction selon la revendication 1 ou 2, **caractérisé en ce que** chaque bras de blocage (**11**) est monté pour s'étendre en relation d'un dégagement (**18**), aménagé axialement sur la vis d'ancrage pour permettre aux bras de blocage d'occuper leur position d'escamotage.

4. Dispositif d'anti-extraction selon l'une des revendications 1 à 3, **caractérisé en ce que** la position de montage des bras de blocage (**11**) sur la vis d'ancrage (**2**) et la position du canal de verrouillage (**28**) sur l'élément d'ostéosynthèse (**3**) sont telles que la vis d'ancrage occupe sensiblement sa position d'introduction finale lorsque les bras de blocage occupent leur position de blocage.

5. Dispositif d'anti-extraction selon l'une des revendications 1 à 4, **caractérisé en ce que** chaque bras de blocage (**11**) comporte un prolongement axial de préhension (**31**), faisant saillie par rapport à la tête (**4**) de la vis d'ancrage.

6. Dispositif d'anti-extraction selon l'une des revendications 1 à 5, **caractérisé en ce que** la tête (**4**) de la vis d'ancrage (**2**) est équipée d'un moyen (**7**) d'entraînement en rotation.

7. Instrument pour le démontage d'une vis d'ancrage (**2**) faisant partie d'un dispositif d'anti-extraction (**1**) conforme à l'une des revendications 1 à 6, **caractérisé en ce qu'**il comporte un manche équipé :
■ d'un moyen de coopération (**42**) avec le moyen (**7**) d'entraînement en rotation de la vis d'ancrage,
■ et d'un moyen de déblocage (**43**) adapté pour exercer un effort radial sur les bras de blocage (**11**), afin de les amener à occuper leur position d'escamotage.

8. Instrument selon la revendication 7, **caractérisé en ce que** le moyen de déblocage (**43**) exerce un effort radial sur les prolongements (**31**) de préhension des bras de blocage.

## Claims

1. An anti-extraction device of an anchoring screw for an osteosynthesis element (3), such a device including:
■ an anchoring screw (2) comprising a head (4) extended with a threaded cylinder (5),
■ and an osteosynthesis element (3) having a bore (21) for letting through the anchoring screw,
**characterized in that**:
■ the anchoring screw (2) at its head, includes at least two blocking arms (11) elastically mounted on the screw, in order to occupy a retracted position under the action of a radial force and a blocking position under the action of a radial force of lower level,
■ and the osteosynthesis element (3) at its bore, includes a radial stress section (23) for the blocking arms, opening into a locking channel (28), downstream considering the engagement direction of the screw, so that during the operation for screwing in the anchoring screw (2), the blocking arms (11) will first occupy their retracted position because of the action exerted by the stress section (23) and then their blocking position when they are placed into the locking channel (28).

2. The anti-extraction device according to claim 1, **characterized in that** each blocking arm (11) consists of a mobile rod (13) attached on the screw (2), extending at rest, substantially parallel to the axis of the anchoring screw and provided with a blocking lug (15) located opposite to the attachment point of the blocking arm.

3. The anti-extraction device according to claim 1 or 2, **characterized in that** each blocking arm (11) is mounted so as to extend in connection with a clearance space (18), axially provided on the anchoring screw to allow the blocking arms to occupy their retracted position.

4. The anti-extraction device according to any of claims 1 to 3, **characterized in that** the mounting position of the blocking arms (11) on the anchoring screw (2) and the position of the locking channel (28) on the osteosynthesis element (3) are such that the anchoring screw substantially occupies its final introduction position when the blocking arms occupy their blocking position.

5. The anti-extraction device according to any of claims 1 to 4, **characterized in that** each blocking arm (11) includes an axial grip extension (31) protruding relatively to the head (4) of the anchoring screw.

6. The anti-extraction device according to any of claims 1 to 5, **characterized in that** the head (4) of the anchoring screw (2) is equipped with a means (7) for driving it into rotation.

7. An instrument for dismounting an anchoring screw (2) being part of an anti-extraction device (1) according to any of claims 1 to 6, **characterized in that** it includes a handle equipped:
■ with a means (42) for cooperating with the means (7) for driving the anchoring screw into rotation,
■ and an unblocking means (43) adapted in order to exert a radial force on the blocking arms (11), in order to cause them to occupy their retracted position.

8. The instrument according to claim 7, **characterized in that** the unblocking means (43) exerts a radial force on the grip extensions (31), of the blocking arms.

## Patentansprüche

1. Vorrichtung gegen das Lösen einer Ankerschraube für ein Osteosyntheseelement (3), wobei eine solche Vorrichtung umfasst:
■ eine Ankerschraube (2), umfassend einen Kopf (4), der durch einen Gewindeschaft (5) verlängert ist
■ und ein Osteosyntheseelement (3), das eine Bohrung (21) zum Durchgang der Ankerschraube aufweist,
**dadurch gekennzeichnet, dass**:
■ die Ankerschraube (2) mindestens zwei Blockierarme (11) auf der Ebene ihres Kopfes umfasst, die an der Schraube in elastischer Art montiert sind, damit sie eine Einfahrstellung unter der Wirkung einer radialen Kraft und eine Blockierstellung unter der Wirkung einer radialen Kraft kleineren Werts einnehmen
■ und das Osteosyntheseelement (3) auf der Ebene seiner Bohrung einen Querschnitt (23) zur radialen Belastung für die Blockierarme umfasst, der abwärts in Eingriffsrichtung der Schraube in einen Verriegelungskanal (28) derart mündet, dass die Blockierarme (11) beim Schraubvorgang der Ankerschraube (2) zuerst ihre Einfahrstellung aufgrund der von dem Belastungsquerschnitt (23) ausgeübten Kraft und dann ihre Blockierstellung unter Platznehmen in dem Verriegelungskanal (28) einnehmen.

2. Vorrichtung gegen das Lösen nach Anspruch 1, **dadurch gekennzeichnet, dass** jeder Blockierarm (11) aus einer bewegbaren Stange (13) besteht, die an der Schraube (2) befestigt ist, indem sie sich in Ruhe im wesentlichen parallel zur Achse der Ankerschraube erstreckt und mit einem Blockiersporn (15) versehen ist, der gegenüber des Befestigungspunkt des Blockierarms angeordnet ist.

3. Vorrichtung gegen das Lösen nach einem der Ansprüche 1 oder 2, **dadurch gekennzeichnet, dass** jeder Blockierarm (11) montiert ist, um sich bezüglich eines Ganges (18) zu erstrecken, der axial auf der Ankerschraube ausgebildet ist, um es den Blockierarmen zu ermöglichen, deren Einfahrstellung einzunehmen.

4. Vorrichtung gegen das Lösen nach einem der Ansprüche 1 bis 3, **dadurch gekennzeichnet, dass** die Montierstellung des Blockierarms (11) an der Ankerschraube (2) und die Stellung des Verriegelungskanals (28) an dem Osteosyntheseelement (3) so sind, dass die Ankerschraube im wesentlichen ihre Endeinführungsstellung einnimmt, wenn die Blockierarme ihre Blockierstellung einnehmen.

5. Vorrichtung gegen das Lösen nach einem der Ansprüche 1 bis 4, **dadurch gekennzeichnet, dass** jeder Blockierarm (11) eine axiale Greifverlängerung (31) umfasst, die in Bezug auf den Kopf (4) der Ankerschraube hervorsteht.

6. Vorrichtung gegen das Lösen nach einem der Ansprüche 1 bis 5, **dadurch gekennzeichnet, dass** der Kopf (4) der Ankerschraube (2) mit einem Mittel (7) zum Drehen versehen ist.

7. Werkzeug zur Demontage einer Ankerschraube (2), welches zu einer Vorrichtung gegen das Lösen (1) gemäß einem der Ansprüche 1 bis 6 gehört, **dadurch gekennzeichnet, dass** es einen Griff umfasst, der versehen ist mit:
■ einem Mittel (42) zum Zusammenwirken mit dem Mittel zum Drehen der Ankerschraube
■ und einem Entblockiermittel (43), das dafür ausgelegt ist, eine radiale Kraft auf die Blockierarme (11) auszuüben, um sie in ihre Einfahrstellung zu bringen.

8. Werkzeug nach Anspruch 7, **dadurch gekennzeichnet, dass** das Entblockiermittel (43) eine radiale Kraft auf die Greifverlängerungen (31) der Blockierarme ausübt.
